(19) Europäisches Patentamt

European Patent Office

Office européen des brevets

(11) **EP 4 706 620 A1**

(12) **EUROPEAN PATENT APPLICATION**

(43) Date of publication:
**11.03.2026 Bulletin 2026/11**

(21) Application number: **25198661.8**

(22) Date of filing: **28.08.2025**

(51) International Patent Classification (IPC):
***A61G 11/00*** *(2006.01)*

(52) Cooperative Patent Classification (CPC):
**A61G 11/005;** A61G 2203/30; A61G 2203/40;
A61G 2203/42

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.09.2024 US 202418828439**

(71) Applicant: **GE Precision Healthcare LLC
Waukesha, WI 53188 (US)**

(72) Inventors:
• **KHAIR, Mohammad
  Waukesha, 53188 (US)**
• **MANICKAM, Kalaivani
  Waukesha, 53188 (US)**
• **FALK, Steven M.
  Waukesha, 53188 (US)**

(74) Representative: **Kilburn & Strode LLP
Lacon London
84 Theobalds Road
Holborn
London WC1X 8NL (GB)**

(54) **INFANT CARE STATION WITH PATIENT SAFETY MONITORING**

(57)    A system for determining a state of an infant care station includes an image sensor through which a first image is captured, a computer processor, and a memory device. The memory device includes instructions executable by the computer processor to determine, based on the first image, a first spatial relationship between a first predetermined point of a first feature that is captured in the first image, and a second predetermined point of a second feature that is captured, and a second spatial relationship between the first predetermined point of the first feature and a third predetermined point of a third feature that is captured. Additionally, the instructions are executable to determine the state of the infant care station based on the first and second spatial relationships. The state of the infant care station indicates whether the walls of the infant care station are latched.

FIG. 1

EP 4 706 620 A1

# Description

## BACKGROUND

The present disclosure generally relates to infant care stations, and more specifically to infant care stations with patient safety monitoring.

Some neonates and prematurely born infants are not physiologically well enough developed to be able to survive without medical attention. A frequently used medical aid for such infants is the incubator. The incubator provides an environment that maintains the neonate at a specific metabolic state, thereby permitting a more rapid physiological development. For example, neonatal incubators create a microenvironment that is thermally neutral where a neonate can develop. Further, these incubators typically include a humidifier and a heater and associated control system that controls the humidity and temperature in the neonatal microenvironment. The humidifier includes a device that evaporates an evaporant, such as distilled water, to increase relative humidity of air within the neonatal microenvironment. The humidifier may control the humidity to a specific percentage by adjusting the amount of water, or water vapor, the humidifier adds to the microenvironment. The heater may be, for example, an air heater controllable to maintain the microenvironment area to a specific temperature. In some scenarios, radiant warmers may be used, instead of incubators, for neonates where less environmental control is specified. In still other embodiments, hybrid incubator/radiant warming systems may be used, various embodiments of which are well known in the art.

## SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

A system for determining a state of an infant care station includes an image sensor through which a first image is captured, a computer processor, and a memory device. The memory device includes instructions executable by the computer processor to determine, based on the first image, a first spatial relationship between a first predetermined point of a first feature that is captured in the first image, and a second predetermined point of a second feature that is captured, and a second spatial relationship between the first predetermined point of the first feature and a third predetermined point of a third feature that is captured. Additionally, the instructions are executable to determine the state of the infant care station based on the first and second spatial relationships. The state indicates whether the walls of the infant care station are latched.

In one embodiment of the infant care station, the first spatial relationship is a first angle, and the second spatial relationship is a second angle.

In one embodiment of the infant care station, the first spatial relationship is a first distance, and the second spatial relationship is a second distance.

In one embodiment of the infant care station, the second distance is represented by a number of pixels between corresponding features of the first image.

One embodiment of the infant care station includes a laser and a laser sensor. The first distance and the second distance are determined using the laser, the laser sensor, and a light detection and ranging method.

In one embodiment of the infant care station, the first feature includes a movable feature, and the first feature is disposed on one of the walls of the infant care station. Further, the second feature is a fixed feature.

In one embodiment of the infant care station, the third feature is a movable feature or a fixed feature.

In one embodiment of the infant care station, the state is determined using a state space model. Further, the state space model uses multiple inputs indicating multiple positions of the first feature, the second feature, and the third feature.

In one embodiment of the infant care station, the instructions include an infant care station interface for obtaining the first spatial relationship and the second spatial relationship. Further, determining the state of the infant care station involves using a machine learning model that is trained to classify the state based on training data comprising multiple spatial relationships corresponding to the first spatial relationship and the second spatial relationship.

One embodiment of the infant care station includes a stereoscopic lens, wherein the image sensor is configured to capture a second image from a different angle than a capture angle of the first image. Further, the first spatial relationship and the second spatial relationship are determined based on a point cloud representing the infant care station. Additionally, the instructions are executable by the computer processor to generate the point cloud based on the first image and the second image.

In one embodiment of the infant care station, the instructions are executable by the computer processor to generate a segmented first image by segmenting the first image. Further, the segmented first image includes multiple representations corresponding to the first feature, the second feature, and the third feature. Additionally, the first spatial relationship and the second spatial relationship are based on the segmented first image.

In one embodiment of the infant care station, the image sensor is disposed within the walls of the infant care station, at a head of the infant care station, outside the walls of the infant care station, and/or at a level that is even with a cover of the infant care station.

In one embodiment of the infant care station, the infant care station is an infant incubator or an infant warmer.

A method for determining a state of an infant care station includes determining, based on a first image of the infant care station that is captured by an image sensor, a first spatial relationship between a first predetermined point of a first feature that is captured in the first image, and a second predetermined point of a second feature that is captured in the first image. Additionally, the method includes determining a second spatial relationship between the first predetermined point of the first feature and a third predetermined point of a third feature that is captured in the first image. Further, the method includes determining the state of the infant care station based on the first spatial relationship and the second spatial relationship. Additionally, the state indicates whether a plurality of walls of the infant care station are latched.

In one embodiment of the method, the first spatial relationship includes a first distance, and the second spatial relationship is a second distance.

In one embodiment of the method, the method includes determining a plurality of positions of the first predetermined point, the second predetermined point, and the third predetermined point by using a light detection and ranging method. Additionally, the method include determining the first spatial relationship and the second spatial relationship based on the plurality of positions.

In one embodiment of the method, the state is determined using a state space model. Further, the state space model uses a plurality of inputs indicating a plurality of positions of the first feature, the second feature, and the third feature.

In one embodiment, the method includes obtaining the first spatial relationship and the second spatial relationship, using an infant care station interface. Additionally, the method includes determining the state of the infant care station by using a machine learning model that is trained to classify the state based on training data comprising a plurality of spatial relationships corresponding to the first spatial relationship and the second spatial relationship. Further, the spatial relationships are associated with multiple infant care stations that are manufactured by multiple different manufacturers.

In one embodiment, the method includes capturing a second image from a different angle than a capture angle of the first image. Additionally, the method includes generating a point cloud representing the infant care station based on the first image and the second image. Further, the method includes determining the first spatial relationship and the second spatial relationship based on the point cloud.

In one embodiment, the method includes generating a segmented first image by segmenting the first image, wherein the segmented first image comprises a plurality of representations corresponding to the first feature, the second feature, and the third feature. Further, the method includes determining the first spatial relationship and the second spatial relationship based on the segmented first image.

Various other features, objects, and advantages of the invention will be made apparent from the following description taken together with the drawings.

BRIEF DESCRIPTION OF THE DRAWINGS

The drawings illustrate the best mode presently contemplated of carrying out the disclosure. In the drawings:

Fig. 1 is a perspective view of an exemplary infant care station with patient safety monitoring according to one embodiment of the present disclosure.
Fig. 2 is a perspective view of an exemplary infant care station with patient safety monitoring according to one embodiment of the present disclosure.
Fig. 3 depicts perspective views of exemplary patient safety monitors according to one embodiment of the present disclosure.
Fig. 4-1 depicts exemplary images of top views of an infant care station with patient safety monitoring according to one embodiment of the present disclosure.
Fig. 4-2 depicts exemplary images of top views of an infant care station with patient safety monitoring according to one embodiment of the present disclosure.
Fig. 5-1 depicts exemplary images of top views of an infant care station with patient safety monitoring according to one embodiment of the present disclosure.
Fig. 5-2 depicts exemplary images of top views of an infant care station with patient safety monitoring according to one embodiment of the present disclosure.
Fig. 6 is a process flow chart of a method for safety monitoring of a patient in an infant care station according to one embodiment of the present disclosure.
Fig. 7 is a diagram of a system for safety monitoring of a patient in an infant care station according to one embodiment of the present disclosure.
Fig. 8 is an exemplary controller for safety monitoring of a patient in an infant care station according to one embodiment of the present disclosure.

DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

The inventors have recognized a problem with current infant care stations-such as incubators, radiant warmers, and other types of neonatal care stations and devices, which is that many infant care stations have walls that can be propped up in such a way that it seems that the walls are latched when they are not. Having a wall of an incubator, for example, in an upright but unlatched position presents a risk that the wall will be inadvertently opened and put a neonate at risk. For example, the neonate could fall out of the incubator if the wall is accidentally opened, or the neonate may be insufficiently protected from impacts or external environmental conditions. While some incubator systems include indicators on latches to indicate when the latch is in a locked position, such indicators can be overlooked by caregivers who may inadvertently allow a wall to remain unlatched while thinking and behaving as if the door is latched and thus secure. Further, even in instances where a caregiver securely latches the infant care station, the neonate may inadvertently kick or punch at the walls of the infant care station, potentially opening one or more walls, or leaving the walls in a state where they are unlatched.

Through significant research and experimentation, the inventors developed the disclosed infant care station which provides improved patient safety by determining when the walls of the infant care station are unlatched, and providing an alert to a care provider, so the care provider may improve the patient safety by securing the unlatched wall(s). More specifically, the disclosed infant care station includes a patient safety monitor with an image sensor that captures one or more images of the infant care station. Further, the patient safety monitor may determine, based on the captured images, whether the walls of the infant care station are latched by determining spatial relationships between features of the infant care station. The spatial relationship can be represented as a distance determined using cartesian coordinates (i.e., x, y, z coordinates). Alternatively, the spatial relationship can be represented using a radial coordinate system with radius and angles. The patient safety monitor may compare the spatial relationships to industry norms and variances. If the spatial relationships fall outside of the industry norms, the patient safety monitor may determine that the walls are unlatched, and provide an alert to a care provider. Advantageously, the patient safety monitor may improve the safety of a variety of infant care stations, produced by a variety of manufacturers.

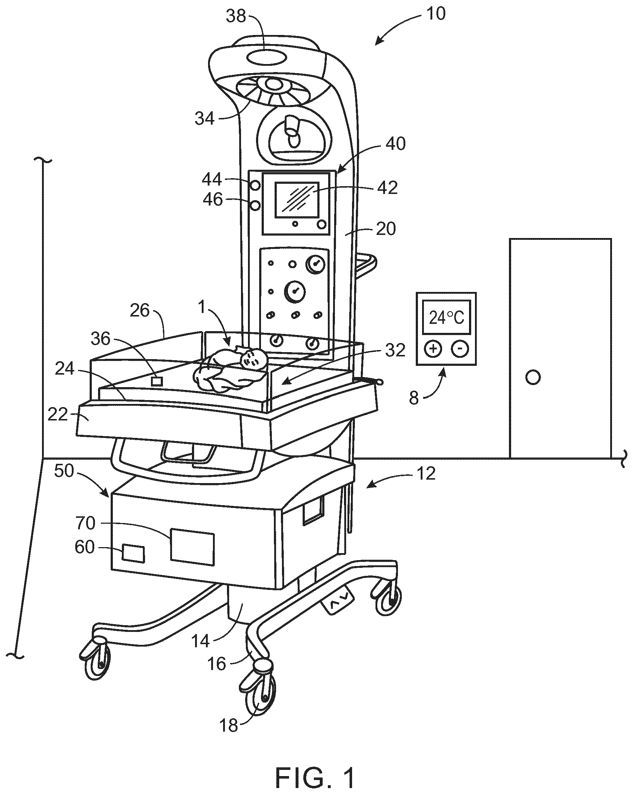

Fig. 1 is a perspective view of an exemplary infant care station 10 with patient safety monitoring according to one embodiment of the present disclosure. The infant care station 10 is shown within a room, such as a labor and delivery suite, or a neonatal intensive care unit, within a medical facility. The ambient air temperature within the room is controlled by room thermostat 8, which is adjustable up and down according to the specification of the patient and medical personnel in a customary manner.

The infant care station 10 shown here is an infant warmer having some elements similar to the Giraffe warmer produced by GE Healthcare™. The infant care station 10 includes a stand 12 supported by legs 14 and feet 16 provided with wheels 18 in a manner presently known in the art. A column 20 extends upwardly from the stand 12 and supports a platform 22. The platform 22 may be height adjustable along the column 20 in a manner presently known in the art. Further, the platform 22 is configured to support a bed 24 (here, a mattress), which is configured to support the patient 1. The walls 26 generally surround and cover the bed 24, to prevent the patient 1 from falling from the bed 24 and also to maintain a controlled environment within the interior. Additionally, the walls 26 may be movable to latch to, unlatch from, each other, thereby providing access to the patient 1. Thus, by latching the movable walls 26 into a secured position, the infant care station 10 can provide a safe, stable, and confined space that may prevent the patient 1 from falling from the bed 24. In some cases, the platform 22 may provide a frame to which the walls 26 and/or cover may latch.

The air within the interior defined by the walls 26 (and when present, the canopy 28) is also referred to as inside air 32. The temperature of the inside air 32 is controlled at least in part by operation of a heater (not shown). The heater may be a heat generating device such as those used within the exemplary warmers described above. It should be recognized that when no canopy 28 is present, the inside air 32 interior is more able to mix with the ambient air within the room. With continued reference to Fig. 1, an enclosure 50 is also supported by the stand 12, and contains a controller 70 for operating the infant care station 10 in a manner presently known in the art. According to one embodiment of the present disclosure, the controller 70 includes a patient safety monitor.

The patient safety monitor may use images to determine whether the walls 26 are latched. Accordingly, the infant care station 10 includes one or more image sensors(s) 38, such as the Intel Realsense® camera, to capture images of at least a portion of the infant care station 10. Further, the image sensor 38 may be positioned on the column 20 such that the image sensor 38 may capture an image of at least three of the walls 26 while the patient 1 is located on the bed 24. Additionally, the image sensor 38 may be sensitive to visible (e.g., red-green-blue [RGB]) and/or infrared light. Further, the image sensor 38 may be sensitive to a light emitting diode (LED) laser. According to one embodiment of the present disclosure, when an illumination level meets or exceeds a predetermined threshold (e.g., brighter conditions), the image sensor 38 may capture images in the RGB spectrum. Alternatively, when the illumination level falls below the predetermined threshold (e.g., darker conditions), the image sensor 38 may capture images in the infrared spectrum. Further, the image sensor 38 may include a lens through which the image(s) is captured. In some embodiments, the lens types may vary in the range of view, and may be a stereoscopic lens, through which the image sensor may capture a stereoscopic image of the infant care station 10 (e.g., two images, each from a different angle). Additionally, the infant care station 10 may include multiple image sensors 38. According to one embodiment of the present disclosure, the image sensor 38 may have two infrared stereoscopic lenses, and a single RGB lens. In such embodiments, the two IR lenses may produce a point-cloud (LiDAR) but detected light texture from an infrared LED (Laser) that spreads a light pattern on the field of view. Further, each of the light dots becomes a point in a point cloud that represents the 3D distances of objects from the lenses.

Further, the patient safety monitor may analyze these images to determine whether the walls 26 are latched. The analysis may be based on spatial relationships between movable features, and/or between movable and unmovable features, of the infant care station 10. A movable feature may be an identifiable portion (e.g., a corner) of a movable wall. The unmovable features may include identifiable portions configured into a fixed position on the infant care station 10. For example, the platform 22 may be configured into a fixed position on the infant care station 10. As such, an edge, corner, or other feature of the platform 22 may represent an unmovable feature.

The spatial relationship may be an angle, or a distance, between the features. For example, the angle may be formed by the edge lines of two walls 26 of the infant care system. Typically, infant warmers are manufactured to have their walls 26 latched in a specific configuration, with respect to the positions of the walls 26 to each other. In a latched state, this angle, across manufacturers of infant warmers, may fall within a relatively small range of seconds of degrees, or smaller. Additionally, the spatial relationship between two specific features may correlate to another spatial relationship between one of these features and a third feature, and/or a spatial relationship between two different features. Accordingly, the patient safety monitor may use linear or non-linear regression techniques to identify correlations between these spatial relationships that indicate whether the walls 26 are latched. For example, a state-space model may be a useful linear regression technique for determining whether the walls 26 are latched. A state-space model is a mathematical model using first order differential equations to represent a real-world, physical system in inputs, outputs, and states. Alternatively, a deep learning neural network model may be a useful non-linear regression technique for determining whether the infant care station 10 is latched. More specifically, it may be possible to train the deep learning neural network to identify a distribution of values (e.g., a specific value and tolerance variations) in spatial relationships that may represent a latched wall. This distribution of values may represent correlations between the spatial relationship measures of the infant care station 10. As such, if the angle formed by these walls 26, and an angle formed by one of these walls 26 and a third wall 26, does not fall within distribution, the patient safety monitor may determine that one or both of the walls are unlatched. With respect to distance, the spatial relationship may be represented by the physical distance between the features on the infant care system 10, or a distance in pixels in the captured image. Thus, the patient safety monitor may compare the distance between features on the infant care system 10 to a distribution of distances between those features for that type of infant care system 10 (e.g., a warmer or incubator). If the comparison reflects a difference that is within a range of tolerance variations for that type of infant care station 10 across manufacturers, the patient safety monitor may determine that the walls 26 are latched. If the difference is outside of the range of tolerance variations, the patient safety monitor may determine that one or more walls 26 are unlatched. Further, the patient safety monitor may use at least one feature on each of at least three walls in view of the image sensor 38 to improve identification of the wall 26 that is unlatched.

The infant care station 10 further includes a user interface 40, which may include a display 42 configured to provide warning indications (text, colors, icons, and the like) as well as messages relating to operation of the infant care station 10. Additionally, the user interface 40 may include a speaker 44, one or more lights 46, and display 42. The speaker 44 and lights 46 may provide further information regarding the operational status of the infant care station 10 and/or communicate information to an operator via sounds, spoken text, spoken words, flashing, varying colors, and/or the lights being on or off. In this manner, as is discussed further below, the user interface 40 provides feedback customary of infant care systems 10 presently known in the art, but also additional information, warnings, and/or the like according to the present disclosure. It should be recognized that the user interface 40 may also or alternatively be provided via an external device (e.g., a mobile device such as a tablet or smart phone) in communication with the infant care system 10. For example, a smart phone may serve as the display 42, speaker 44, and/or lights 46 (alone or in conjunction with another display 42, speaker 44, and lights 46, on the infant care station 10) that communicates with the infant care station 10 via Bluetooth® or another wireless protocol known in the art. Further, according to one embodiment of the present disclosure, the display 42, speaker 44, and lights 46, may provide a warning or other indication that the walls 26 are latched or unlatched. For example, if the patient safety monitor determines that all the walls 26 are latched, the patient safety monitor may direct the display 42 to show a message indicating the walls 26 are latched. Additionally, or alternatively, the patient safety monitor may direct one of the lights 46 representing safety (or, a latched state) to illuminate in a green color to indicate the latched state. Conversely, if the patient safety monitor determines that one or more walls 26 are unlatched, the patient safety monitor may direct the speaker 44 to sound an alarm, or a voice stating the walls are unlatched. Additionally, the patient safety monitor may direct the display 42 to show a flashing message stating which walls 26 are unlatched, and/or direct one or more lights 46 to illuminate in a red color and/or a flashing pattern.

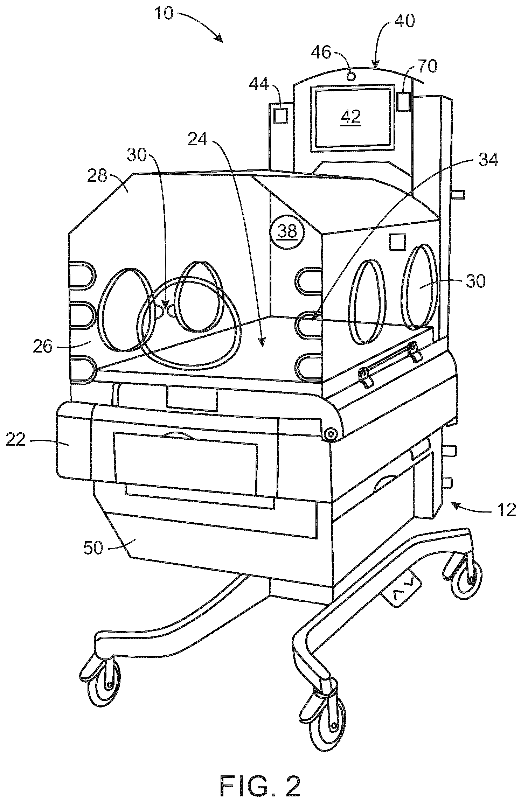

Fig. 2 is a perspective view of an exemplary infant care station 10 with patient safety monitoring according to one embodiment of the present disclosure. In this example, the infant care station 10 is similar to that of Fig. 1, but now as an incubator rather than an infant warmer. Similar to Fig. 1, the infant care station 10 of Fig. 2 includes stand 12, platform 22, bed 24, walls 26, openings 34, image sensor 38, user interface 40, enclosure 50, and controller 70. The openings 34 may be pass-through openings for passing cables, tubes, and the like into the incubator. Additionally, the infant care station 10 of Fig. 2 includes a canopy 28, whereby the interior of the infant care station 10 is defined by the walls 26 and the canopy 28. Further, the incubator of Fig. 2 includes portholes 30 within the walls 26 and/or canopy 28 to provide access to the interior (e.g., patient 1, bed 24, and/or the platform 22) without opening one or more of the walls 26 and/or the canopy 28 in a manner presently known in the art.

The canopy 28 can be elevated from, and lowered onto the four walls 26. Further, the configuration of the walls 26 and cover may vary in different embodiments of the present disclosure. For example, the infant care station 10 may have four walls 26, with a canopy 28 that is positioned atop the four walls. Alternatively, the canopy 28 may include a piece that extends into the spaces occupied by one or more of the walls 26 in Fig. 1. In such an embodiment, the infant care station 10 may include two or three walls 26, with the extended cover providing the wall structure in the spaces unoccupied by the canopy 28. Further, the walls 26 may be fixed to each other, the canopy 28, and/or the platform 22.

The image sensor 38 and controller 70 of Fig. 2 may be similar to the image sensor 38 and controller of Fig. 1. As such, the controller 70 may include a patient safety monitor that may determine the latched or unlatched state of the infant care system 10 by analyzing one or more images captured by the image sensor 38. Accordingly, the patient safety monitor may determine spatial relationships between features of the incubator, such as the portholes 30 on different walls 26, openings 34, and the edge of the platform 22. Thus, the patient safety monitor may determine the distances between a porthole 30 on one of the sidewalls to a second porthole on the canopy 28, and to a third porthole on the opposing side wall. Further, the patient safety monitor may compare the distances to a distribution of distances between those features for incubators produced by various manufacturers. If the comparison reflects a difference that is within a range of tolerance variations for that type, the patient safety monitor may determine that one or more of the walls 26 are latched. If the difference is outside of the range of tolerance variations, the patient safety monitor may determine that one or more walls 26 are unlatched.

Accordingly, if the patient safety monitor determines that the walls 26 are latched, the patient safety monitor may direct the display 42 to show a message indicating the walls 26 are latched. Additionally, or alternatively, the patient safety monitor may direct one of the lights 46 to illuminate in a green color to indicate the walls 26 are latched. Conversely, when the patient safety monitor determines that one or more walls 26 are unlatched, the patient safety monitor may direct the speaker 44 to sound an alarm. Additionally, the patient safety monitor may direct the display 42 to show a flashing message that the walls 26 are unlatched, and/or direct one or more lights 46 to illuminate in a red color and/or a flashing pattern.

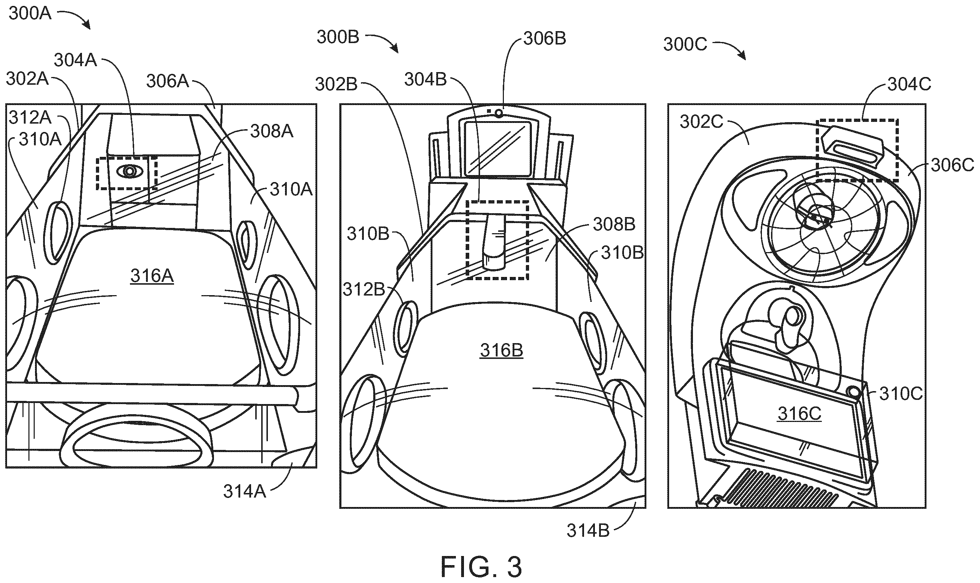

Fig. 3 depicts perspective views of exemplary systems 300A, 300B, 300C (collectively referred to as systems 300) for patient safety monitors according to one embodiment of the present disclosure. The systems 300 represent several implementations of exemplary infant care stations 302A, 302B, 302C (collectively referred to as infant care stations 302), image sensors 304A, 304B, 304C (collectively referred to as image sensors 304), and stands 306A, 306B, 306C (collectively referred to as stands 306). The stations 302, image sensors 304, and stands 306 may be similar to the infant care stations 10, image sensors 38, and stands 12 (and columns 20) described with respect to Figs. 1, 2. Further, the infant care stations 302 have walls 310A, 310B, 310C (collectively referred to as walls 310), in a rectangular configuration around beds 316A, 316B, 316C (collectively referred to as beds 316), upon which the patient 1 lies, or otherwise reclines. The infant care stations 302 include incubators 302A, 302B and an infant warmer 302C. For the purpose of this description, the walls 310 are referred to as a north, south, east and west configuration, with the front wall (e.g., closest to the viewer of Fig. 3) representing the south, the side walls (from left to right) representing east and west respectively, and the back wall (furthest from the viewer of Fig. 3) representing the north. The walls 310A, 310B of the incubators 302A, 302B have portholes 312A, 312B (collectively referred to as portholes 312). Additionally, the incubators 302A, 302B include covers 308A, 308B (collectively referred to as covers 308), and utility openings 314A, 314B. The covers 308 (e.g., canopies) and portholes 312 may be respectively similar to the canopy 28 and portholes 30 described with respect to Fig. 2. The utility openings 314A, 314B may provide a passage for wires and/or tubing that is connected with external equipment that may facilitate monitoring and/or providing assistance and/or sustenance to the patient 1.

With respect to the system 300A, the image sensor 304A may be positioned approximate to the north wall of the incubator 302A. Further, the system 300B includes a stand 306 having a display 318, speaker 320, and lights 322 similar to the display 42, speaker 44, and lights 46 described with respect to Fig. 2. Additionally, the system 300B includes an image sensor 304B incorporated with, or otherwise connected with, the incubator 302B, and positioned above the bed 316. The image sensor 304B may be positioned in a way that mitigates reflection off the cover 308 during image capture. For example, the image sensor 304B may be located on, or relatively near, the cover Further, the infant warmer 302C includes an image sensor 304C incorporated or otherwise connected to the stand 306C and positioned above the bed 316C and walls 310C.

According to one embodiment of the present disclosure, the systems 300 may include more than one image sensor 304. Additionally, the image sensors 304 may be located within the walls 310, incorporated with the walls 310, or incorporated with the stands 306 and/or some other device. It is noted that the positions of the image sensors 304 are merely examples, and embodiments of the present disclosure may have the image sensors 304 located in other positions. The positions may provide the image sensors 304 a view such that images captured by the image sensor 304 (while the patient 1 is on the bed 316) have a relatively unobstructed view of at least three walls 310 of the infant care stations 302. Further, according to one embodiment of the present disclosure, the image sensors 304 may capture images from one or more angles of this view. The captured images may be in RGB and/or infrared spectrums, and may be captured through various lenses providing specific range(s) and angle(s) of viewing. Additionally, the image sensors 304 may be calibrated such that it is possible to determine distances between features on the walls 310 to at least sub-millimeter, or smaller, accuracy, based on the positions of pixels representing those features in one or more images, and the position of the image sensors 304.

More specifically, embodiments of the present disclosure may identify three or more feature positions (e.g., specific points on three or more walls 310), and determine a spatial relationship (e.g., distance and/or angle) between these positions. When latched, these distances and/or angles may vary by relatively small amounts (e.g., a sub-millimeter of distance, a sub-second of an angle), regardless of manufacturer of the infant care system 302. Accordingly, it is possible to collect data identifying the spatial relationships between various feature positions on incubators or infant warmers of numerous manufacturers, and train a deep learning neural network machine learning model using this data to determine whether the spatial relationships between these feature positions in exemplary infant care stations 302 fall within a particular distribution of measurements and tolerance variations. If so, a controller (e.g., patient safety monitor) can determine the spatial relationships between predetermined features on an exemplary infant care station 302, and use such a trained model (e.g., a latched state model) to classify the infant care station 302 as latched or unlatched. Accordingly, the patient safety monitor may provide an indicator that the infant care station 302 is latched, or if not, sound an alarm, produce images on a monitor, and/or illuminate lights in various colors and flashing patterns to attract the attention of a caretaker who may securely latch the infant care station 302. Further, in the case of false positives and false negatives, the patient safety monitor may provide training data to improve this machine learning model prediction or classification accuracy. Deep neural networks are efficient nonlinear relationship regression tools that can be used for prediction or classification. As stated previously, the patient safety monitor may alternatively use state-space models for generalized linear regression to determine if the walls 310 are latched. The use of linear versus non-linear models may depend on the type of input data used for modeling the position of the walls, or the latched state of these walls. For input data that is of a single type, e.g., representing spatial distances in cartesian coordinates (e.g. absolute or relative distances), a linear model may be useful. However, for input data that includes a combination of cartesian and polar coordinate system measurements, a deep neural network to model non-linear relationships, e.g., sine or cosine of an angle, may be useful.

According to one embodiment of the present disclosure, the trained model may classify walls 310 as latched or unlatched by determining a probability that each of the walls 310 is latched based on the training data. Accordingly, the patient safety monitor may determine whether the incubator is latched based on these probabilities. For example, when one or more, and/or combination of, probabilities meet one or more predetermined thresholds, the patient safety monitor may determine that the incubator is latched. Alternatively, the patient safety monitor may determine one or more incubator walls are unlatched. Additionally, the patient safety monitor may identify the wall(s) as unlatched that do not meet these thresholds. Further, the patient safety monitor may take an action, as described above.

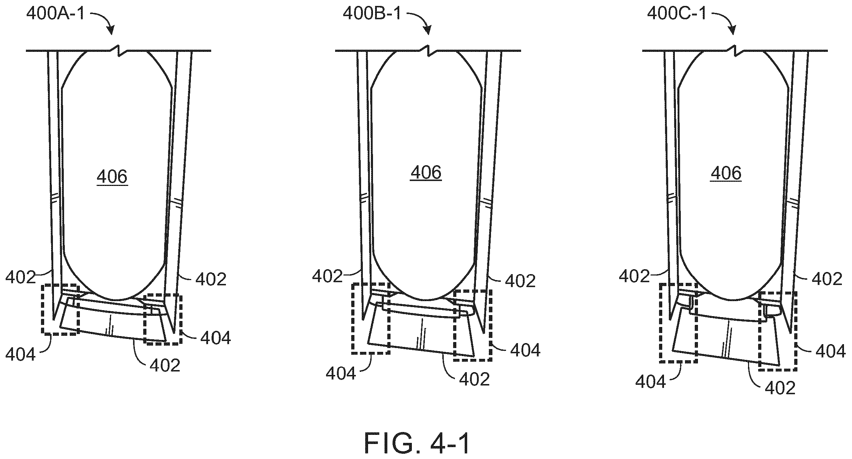

Fig. 4-1 depicts exemplary images 400A-1, 400B-1, 400C-1 (collectively referred to as images 400-1) of top views of an infant care station with patient safety monitoring according to one embodiment of the present disclosure. In this example, the images 400-1 may represent images captured by the image sensor 304C, positioned over the infant warmer 302C, as described with respect to Fig. 3. Further, the images 400-1 show the infant warmer in various states of being latched and unlatched. As the images 400-1 show, the infant warmer includes walls 402 around a bed 406. Between the southern wall, and each of the east and west walls, there is a gap 404. According to one embodiment of the present disclosure, the gaps 404 may be used as a feature to determine the latched state of the infant warmer. For example, the size of the gaps 404, distances across the gaps 404, and the like, may be indicative of various states of the walls 402 being latched, or unlatched. In this example, the gaps 404 increase in size (and distance) from left to right, i.e., the gaps 404 in image 400A are smaller than the gaps 404 in image 400B, which are smaller than the gaps 404 in image 400C. Accordingly, in image 400A-1, the walls 402 of the infant care station may be latched. However, in the images 400B-1, 400C-1, one or more of the walls 402 may not be latched.

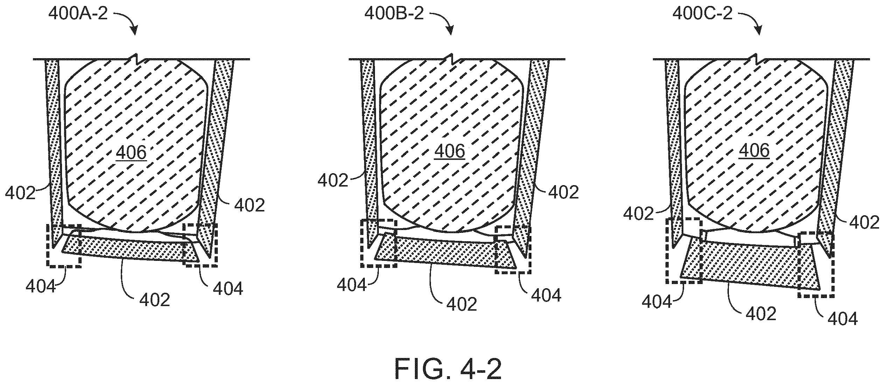

Fig. 4-2 depicts exemplary segmented images 400A-2, 400B-2, 400C-2 (collectively referred to as segmented images 400-2) of the infant warmer shown in images 400-1 according to one embodiment of the present disclosure. The segmented images 400-2 may represent modified versions of the images 400-1, wherein the segmented images 400-2 may include modifications that identify features of the images 400-1. In this example, the modifications are hash marks, but various embodiments may use different visual markings, such as color, shadings, and the like. In some embodiments of the present disclosure, the patient safety monitor may use a machine learning model trained to segment images into component features. One example of such a machine learning model is Meta AI's Segment Anything Model (SAM). Accordingly, the patient safety monitor may use the segmenting machine learning model to analyze the pixels of the images 400-1 and identify contiguous regions that may represent distinct objects (e.g., features of the infant care system) in the images 400-1. These identified regions can be output as image masks of the objects segmented in the field of view. Further, this model, or other software using this model, may visually segment the image 400-1 into one or more identified features. Accordingly, such software may uniformly color, pattern, or otherwise visually identify contiguous regions on the images 400 to indicate one or more distinct features. In this example, segmented images 400-2 identify the walls 402 and beds 406 as segmented features. More specifically, the patient safety monitor may, for each identified feature, use its maximum (or minimum) x or y coordinate position in image pixels. Further, the patient safety monitor may use the calibrated image produced by the image sensor 38 with depth imaging (stereo IR point-cloud) to find the position of the equivalent pixel with x,y,z point cloud coordinate values.

Accordingly, the patient safety monitor may use the positions of the segmented features (feature positions) in the segmented images 400-2 to determine the spatial relationships between the feature positions. For example, the patient safety monitor may determine positions of the corners of the walls bordering the gaps 404 (i.e., the corners of the walls 402 at the southern end of the infant warmer). Further, the patient safety monitor may determine the distances between these corners. The controller may also determine a size (i.e., area in square millimeters [mm's]) of the gaps 404. Alternatively (or additionally), the patient safety monitor may determine the angles formed by the lines representing the tops (or bottoms, or sides) of the walls 402, and the lines formed by the edges of the bed 406, or platform. Further, the patient safety monitor may use Cartesian or polar geometry to determine these spatial relationships. Accordingly, the patient safety monitor may use the machine learning model trained to determine the probabilities as to whether walls of an infant care station are latched, to determine these probabilities for the infant warmers shown in segmented images 400-2, based on the spatial relationships represented by the gaps 404, and/or the angles described above.

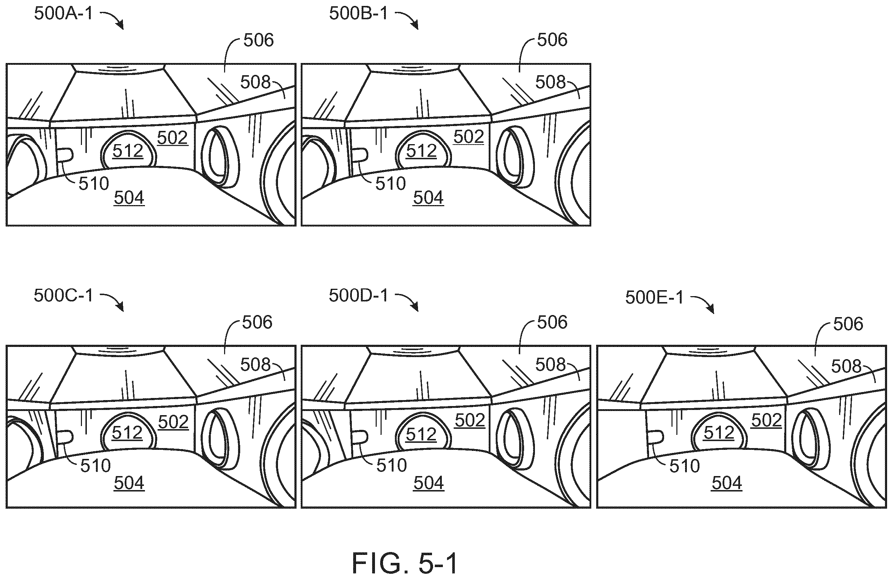

Fig. 5-1 depicts exemplary images 500A-1, 500B-1, 500C-1, 500D-1, 500E-1 (collectively, images 500-1) of an infant care station with patient safety monitoring according to one embodiment of the present disclosure. In this example, the infant care station is an incubator, similar to the incubator 302A, described with respect to Fig. 3. The incubator has walls 502 surrounding a bed 504. Additionally, the incubator has a cover 506 positioned over the walls 502, and delineated by a rim 508 that runs atop the walls 502. Similar to the walls 310A, 310B of incubators 302A, 302B described with respect to Fig. 3, the walls 502 include a utility hole 510 and portholes 512. In this example, the images 500-1 represent images captured from an image sensor, positioned similarly to the image sensor 304A. Additionally, the images 500-1 show an incubator in various states of being latched and unlatched. More specifically, the image 500A-1 represents an incubator in a latched state. Further, the successive images 500B-1, 500C-1, 500D-1, 500E-1, represent the incubator with the eastern wall growing further open. More specifically, in the image 500E-1, the eastern wall is so far open that the corresponding porthole 512 is no longer visible.

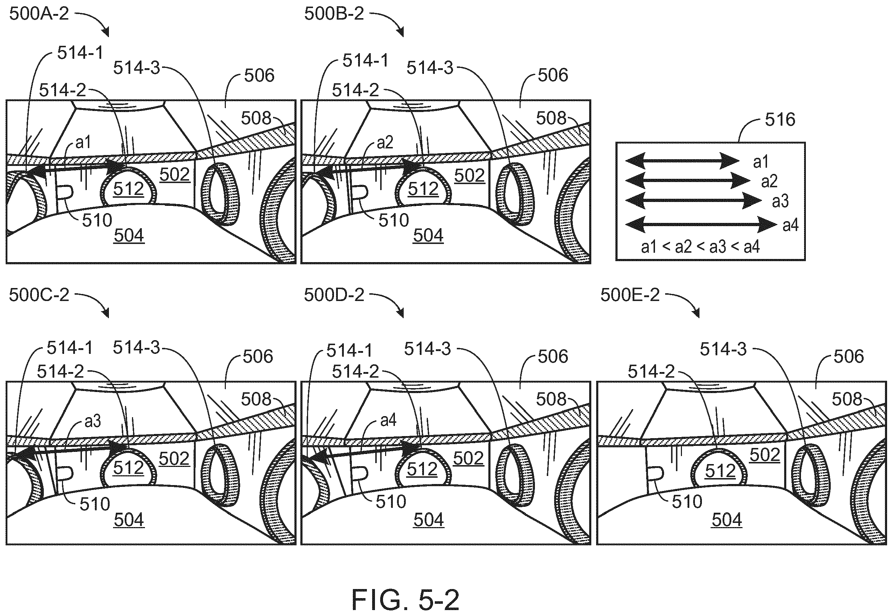

Fig. 5-2 depicts exemplary segmented images 500A-2, 500B-2, 500C-2, 500D-2, 500E-2 (collectively, segmented images 500-2) of the images 500-1 and distance indicators a1, a2, a3, a4 according to the present disclosure. Similar to the images 400-1 and segmented images 400-2 described with respect to Figs. 4-1, 4-2, the segmented images 500-2 represent the images 500-1 modified to identify features. As stated previously, the patient safety monitor may use a machine learning model, such as an image segmentation model (e.g., Meta's SAM model) trained to segment the mages 500-1 into component features of the infant care system. In this example, the identified segments include the walls 502, bed 504, cover 506, rim 508, utility hole 510, and portholes 512. Further, the patient safety monitor may identify predetermined feature positions of the infant care stations. For example, the predetermined feature positions may be some combination of feature positions 514-1, 514-2, 514-3 on the portholes 512, a feature position (e.g., a predetermined corner) on the utility hole 510, or some other predetermined position on the cover 506, cover rim 508, and/or any other features indicated by the segmentation. According to one embodiment of the present disclosure, the patient safety monitor may use segmentation to determine equivalent mask (binary 0 or 1) images for each of the segmented regions. Further, the patient safety monitor may use these masks to extract the section of the calibrated image produced by a projection of the point-cloud produced by the depth sensor. This point cloud has calibrated x,y,z positions for the all objects in the field of view, and by applying the mask, it is possible to extract the segmented region x, y, z coordinates. Accordingly, the x, y, z coordinates may inform the position, size, and orientation of the feature in three-dimensional (i.e., x, y, z) space.

Similar to image 500A-1, the segmented image 500A-2 may represent the incubator in a latched state. Conversely, the segmented images 500B-2, 500C-2, 500D-2, 500E-2 may represent an infant care station in advanced degrees of openness. These degrees of openness are represented in the lengths of the distance indicators a1, a2, a3, a4. More specifically, the distance indicators represent the distance between feature position 514-1 on the east wall and feature position 514-2 on the south wall. As shown in the legend 516, the distance indicator a1 is smaller than distance indicator a2, which is smaller than distance indicator a3, which is smaller than indicator a4. According to one embodiment of the present disclosure, the controller may provide these distances to the latched state model to determine the probabilities that the walls 502 are latched. Further, in segmented image 500E-2, the eastern wall is open. As such, the corresponding porthole is not visible. Accordingly, the patient safety monitor may use the segmented image 500E-2 (and not the trained model) to determine that because the porthole is not visible, the wall is open, and hence, unlatched.

As shown in the legend 516, the distance lines grow successively longer with respect to the distances indicated in segmented images 500A-2 through 500D-2. While not shown, the patient safety monitor may also determine the distances between each of the feature positions 514-1, 514-2, and feature position 514-3. In this way, the patient safety monitor may also use the latched state model to determine whether the western and southern walls are latched or unlatched. Based on these determinations, the patient safety monitor may determine whether only one of the walls 502 is unlatched, or some combination.

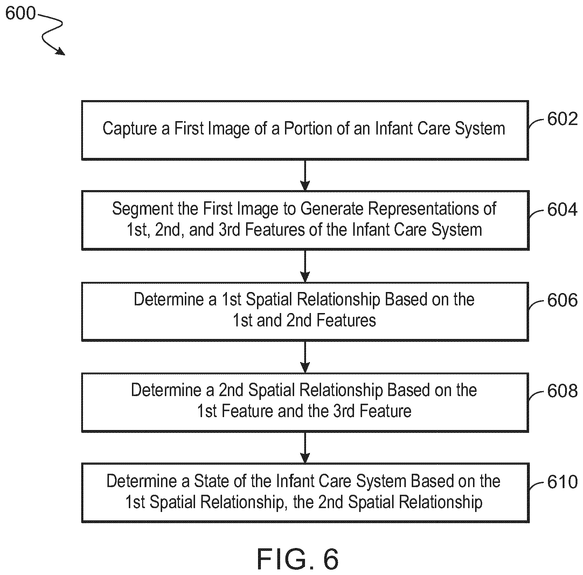

Fig. 6 is a process flow chart of a method 600 for safety monitoring of a patient in an infant care station according to one embodiment of the present disclosure. The patient safety monitor, described with respect to Figs. 1-5, may perform the method 600 according to one embodiment of the present disclosure.

At operation 602, the patient safety monitor may direct one or more image sensors to capture an image of an infant care system. The image sensor(s) may be similar to the image sensors 38, 304, described with respect to Figs. 1-3. The image capture may be in the RGB and/or infrared spectrum. In some embodiments, the controller may select the spectrum for capture based on an illumination level of the environment of infant care system (such as whether the room where the infant care system is located is illuminated or is dark). As such, if the illumination level meets or exceeds a predetermined threshold, the patient safety monitor may direct the image sensor(s) to capture the image(s) in the RGB spectrum. If the illumination does not meet the predetermined threshold, the patient safety monitor may direct the image sensor to capture the image(s) in the infrared spectrum.

At operation 604, the patient safety monitor may segment the captured image(s) to identify at least first, second, and third features. According to one embodiment of the present disclosure, the patient safety monitor may provide the captured image(s) to a machine learning model to segment the images. For example, the patient safety monitor may provide the captured image(s) to the segmentation model. Further, the segmentation model (e.g., the SAM) may provide a segmented image with masks for each identified segment.

At operation 606, the patient safety monitor may determine a first spatial relationship based on the first and second features. If the spatial relationship to be determined is an angle between features, the patient safety monitor may compare the relative positions of the first and second features, and determine an angle of incidence between them. If the spatial relationship to be determined is a distance between features, the patient safety monitor may identify a position in the segmented image for each of the first and second features. For example, if the first and second features are the apexes of a porthole on a west wall and a porthole on the east wall, the controller my identify the pixels in the segmented image(s) where the apex is represented. If the distance to be determined is a pixel distance, the patient safety monitor may determine the distance based on the pixel coordinates of each of the features. However, if the distance to be determined is a physical distance between the features, the patient safety monitor may use a laser distance imaging and ranging technique called Light Detection and Ranging (LIDAR) method whereby artificial light texture is added to the field of view to determine the locations of each of the features in physical space in the image view and their depth, and form a point-cloud graphical representation of these features in 3D. Further, the patient safety monitor may determine the distance between the features based on the locations determined.

According to one embodiment of the present disclosure, the patient safety monitor may determine the physical location of the features and feature positions by generating a depth point cloud of the captured image. In such an embodiment, the image sensor may capture a stereoscopic image of the infant care station. The stereoscopic image consists of two images of the infant care station taken at slightly different angles. Accordingly, the depth camera may compare the depth (i.e., distance from the image sensor) of each pixel in one of the images to depth of the corresponding pixel in the other image generating an x, y, and z position in 3D for each pixel in the field of view where the x and y indicate the calibrated position and the z indicates the calibrated depth from the camera. These x,y,z points are calculated at the points of a scattered artificial light texture added to the scene of view using an IR laser (LED) with a scattering light pattern. A point-cloud can therefore be generated for each of the dots of the scattered light pattern representing this artificial light texture, which represents the 3D location for each feature in the field of view. Further, the patient safety monitor may determine the actual distance of the feature captured in the pixel based on the comparison, and the angle formed by the lens of the image sensor and the feature captured in the pixel.

At operation 608, the patient safety monitor may determine a second spatial relationship based on the first and third features. Determining the second spatial relationship is similar to determining the first spatial relationship, as described above. However, the location of the third feature and/or feature position may be determined instead of those of the second feature.

At operation 610, the patient safety monitor may determine a state of the infant care system based on the first and second spatial relationships. According to one embodiment of the present disclosure, the patient safety monitor may provide the first and second spatial relationships to a latched state model. Further, the latched state model may determine, based on the spatial relationships, a probability as to whether is each of the walls represented in the spatial relationships is latched, and provide these probabilities to the patient safety monitor. Additionally, the patient safety monitor may determine whether each of the walls is latched based on whether the determined probabilities meet predetermined thresholds. If the probability does not meet the predetermined threshold, the patient safety monitor may determine that the state of the wall is unlatched. Conversely, if the probability does meet the predetermined threshold, the patient safety monitor may determine that the state of the wall is latched.

According to one embodiment of the present disclosure, the deep learning model (i.e., deep learning neural network) may use generalized non-linear regression or classification. Generalized non-linear regression may provide a prediction function that can be thresholded for determining whether the walls are latched. Conversely, classification may provide a likelihood of one or more states (classes), i.e., a probability, that the walls are latched and probability that walls are unlatched. A deep learning neural network includes an input first layer having multiple nodes, multiple hidden layers, and an output final layer. Each node applies an activation function to the inputs, the output of which indicates the inputs to the next layer. According to one embodiment of the present disclosure, the activation function may be a rectified linear unit (ReLU). Other activation functions such as sigmoid or tanh can be used as well. Each hidden layer processes the outputs from the previous layer, and subsequent layers halve the number of nodes from the previous layer until the final layer provides the output. The final layer may have one or more nodes, depending on the type of output the model is providing. For example, a single node final layer may represent a prediction output, e.g., indicating whether the walls 310 of the infant care station 302 are latched. In such an embodiment, values closer to 0 may indicate an unlatched state, and values closer to 1 may indicate a latched state. Where the output layer includes multiple nodes, an output layer having two nodes for prediction may provide an x and y coordinate of a feature position. In such a case, the x, y position can indicate a planar top view of a wall position relative to the camera coordinate system. As such, it may be possible to determine the latched state by thresholding the absolute x, y location. Further, a final layer having three nodes for prediction may provide three coordinates, e.g., x, y, and z coordinates, indicating the position of a feature. In such a case, the x, y, z position can indicate a 3D view of a wall position relative to the camera coordinate system, As such, it may be possible to determine the latched state by thresholding the absolute x, y, and z location. Alternatively, for classification, an output layer may use a softmax layer that outputs a probability numeric value between 0 and 1 for each of the output classes (latched class and unlatched class), where the sum of all probabilities for all classes is 1. For classification a two node output layer may provide two probabilities (that sum to 100%) of two exclusive conditions, e.g., a probability of the walls being latched and a probability of the walls being unlatched.

Alternatively, instead of using a deep learning neural network model to determine the latched or unlatched state, the patient safety monitor may use a state-space model. A state-space model may be a useful linear regression technique for determining whether the walls 26 are latched. A state-space model is a mathematical model using first order differential equations to represent a real-world, physical system in inputs, outputs, and states (over time). In continuous-time, a state-space model is of the following form, as shown in SYTEM OF EQUATIONS 1.

$$x' = Ax + Bu$$
$$y = Cx + Du$$
SYSTEM OF EQUATIONS 1

Here, x, u and y represent the states, inputs and outputs respectively. Additionally, A, B, C and D are the state-space coefficients matrices. The x' represents the derivative of the state x. The state represents the unforced dynamics of the system after the system is exposed to a unit pulse as an input u. States therefore can carry the history of dynamic changes of the system due to inputs presented to it. Accordingly, the inputs can represent the spatial relationships of the infant care system, and the output can indicate a function that can be thresholded to determine whether the walls are latched.

In discrete-time, the state-space model can be represented as shown in SYSTEM OF EQUATIONS 2.

$$X[t+1] = Ax[t] + Bu[t]$$
$$Y[t] = Cx[t] + Du[t]$$
SYSTEM OF EQUATIONS 2

In the SYSTEM OF EQUATIONS 2, t represents the current time step. Accordingly, future states at time-step t+1 are calculated from previous states x[t] and inputs u[t] at time step t, while current output y[t] is calculated from the current state x[t] and current input u[t]. The inputs can represent the one or more of the two-dimensional (2D) x, y distances, pixel distances, and/or 2D orientation angles between several features in the field of view that may model the relationships of the inputs, u, to the outputs, y. Alternatively, The inputs can represent the one or more of the three-dimensional (3D) x, y, and z distances, pixel distances, and/or 3D orientation angles between several features in the field of view that may model the relationships of the inputs, u, to the outputs, y. According to one embodiment of the present disclosure, the outputs, y, as well as the inputs, u, can be vectors representing distances or angles representing measured distances from complex geometrical relationships between the input variables. The adaptive nature of the state-space model can be a useful technique to model such complex relationships between the inputs and the outputs. Alternatively, the output vector can also be 0 or 1 boolean for latched or unlatched state of the walls. According to another embodiment of the present disclosure, the output vector, y, can be a numeric value in the range of 0 to 1, whereby the patient safety monitor may use a predetermined thresholds in this range to determine whether the walls are latched or unlatched.

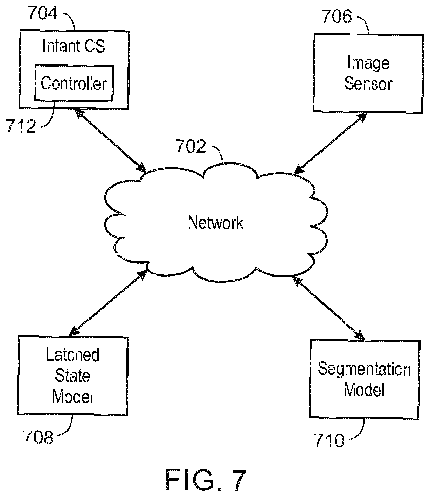

Fig. 7 is a diagram of a system 700 for safety monitoring of a patient in an infant care station according to one embodiment of the present disclosure. The system 700 may include a network 702, infant care station 704, image sensor 706, latched state model 708, and segmentation model 710. The network 702 may be a computer communication network or collection of networks, such as a local area network, wide area network, and the like. In some embodiments of the present disclosure, the network 702 is the Internet. Accordingly, the infant care station 704, image sensor 706, latched state model 708, segmentation model 710, and elements therein, may communicate with each other over the network 702.

The infant care station 704 may be similar to the infant care stations 10, 302, described with respect to Figs. 1-6. Further, the image care station 704 may include a controller 712, which may be similar to the patient safety monitor described with respect to Figs. 1-6. Additionally, the image sensor 706 may be similar to the image sensors 38, 304 described with respect to Figs. 1-3. Further, the latched state model 708 may be a machine learning model similar to the latched state model described with respect to Figs. 1-6. Further, the segmentation model 710 may be a machine learning model similar to the SAM model described with respect to Figs. 4-2, 5-2. Further, the latched state model 708 and segmentation model 710 may run on one or more server devices.

According to one embodiment of the present disclosure, the controller 712 may direct the image sensor 706 to capture images of the infant care station 704. Additionally, the controller 712 may use the segmentation model 710 to identify features captured in the images. Further, the controller 712 may identify feature positions on the identified features and determine spatial relationships between the identified feature positions. Additionally, the controller 712 may use the latched state model 708 to determine the probabilities that each of the walls of the infant care station 704 is latched. Further, the controller 712 may determine whether each of the walls is latched based on these probabilities. Additionally, the controller 712 may provide a visual, audio, and/or other sensory indicator of the latched state. The controller 712 may provide such indicators on a local device, such as the user interface 40 described with respect to Fig. 2 and/or a mobile device, such as a smartphone.

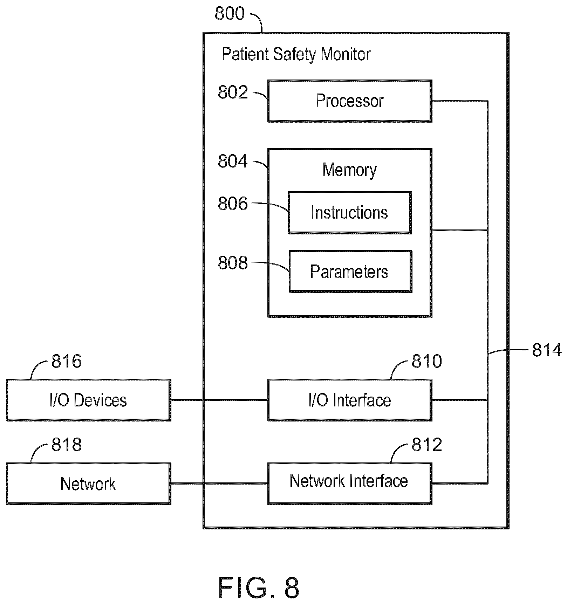

Fig. 8 is a diagram of an example controller 800 according to the present disclosure. The example controller 800 may monitor the spatial relationships between the walls of an infant care station, and provide indications of the latched or unlatched state of the walls, as described with respect to Figs. 1-7. In this example, the controller 800 includes a processor 802, memory 804, input-output (I/O) interface 808, and network interface 810, which may be connected by an interconnect 812. The processor 802 may be a computer processing circuit (e.g., a central processing unit (CPU)) that retrieves and executes programming instructions 806 stored in the memory 804 to perform the functionality described herein. The interconnect 812 may move data, such as programming instructions, between the processor 802, memory 804, I/O interface 808, and network interface 810. The interconnect 812 may include one or more buses.

The memory 804 may be a computer memory or storage device, including volatile memory, such as a random access memory (RAM) device (e.g., static RAM, dynamic RAM, and the like), non-volatile memory, such as a hard disk drive, solid state device (SSD), removable memory cards, optical storage, flash memory devices, and the like. In some examples, the memory 804 may include volatile and non-volatile memory devices. Further, the memory 804 may store parameters 805 and instructions 806. The parameters 805 may be useful for determining distances based on triangulation, and for making determinations about the latched or unlatched states of the walls of infant care stations (e.g., the probability thresholds described above). For example, the parameters 805 may identify the feature positions, the position(s) of the image sensor(s), and instructions 806.

Additionally, the controller 800 may be in electronic communication with I/O devices 814 through the I/O interface 808, and with a network 816 through the network interface 810. The I/O devices 814 may capture inputs and provide outputs as described herein. More specifically, the image sensors 38, 304, described with respect to Figs. 1-3 may be input devices. Additionally, the display 42, speaker 44, and lights 46 described with respect to Figs. 1-3 may be the output devices. The network 816 may be an electronic communication network, such as a local area network, wide area network, and the like, for processing communications between the controller 800 and the machine learning models and AI software products described herein. In some examples, the network 816 may be wired, wireless (e.g., wi-fi, Bluetooth, or cellular), or some other computer communication network.

In some embodiments, the controller 800 may be a server computer or similar device without a user interface but which receives requests from other computer systems having one or more user interfaces. Further, in some embodiments, the controller 800 may be a portable computer, laptop, tablet computer, pocket computer, telephone, smart phone, or the like.

As used herein, the term, mechanism, can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1.   A system for determining a state of an infant care station, comprising:

an image sensor through which a first image is captured;
a computer processor; and
a memory device comprising instructions executable by the computer processor to:

determine, based on the first image:

a first spatial relationship between a first predetermined point of a first feature that is captured in the first image, and a second predetermined point of a second feature that is captured in the first image; and
a second spatial relationship between the first predetermined point of the first feature and a third predetermined point of a third feature that is captured in the first image; and

determine the state of the infant care station based on the first spatial relationship and the second spatial relationship, wherein the state of the infant care station indicates whether a plurality of walls of the infant care station are latched.

2.   The system of claim 1, wherein the first spatial relationship comprises a first angle, the second spatial relationship comprises a second angle.

3.   The system of claim 1, wherein the first spatial relationship comprises a first angle, the second spatial relationship comprises a distance.

4.   The system of claim 1, wherein the first spatial relationship comprises a first distance, and wherein the second spatial relationship comprises a second distance.

5. The system of claim 4, wherein each of the first distance and the second distance is represented by a number of pixels between corresponding features of the first image.

6. The system of claim 5, comprising a laser and a laser sensor, wherein the first distance and the second distance are determined using the laser, the laser sensor, and a light detection and ranging process.

7. The system of claim 1, wherein the first feature comprises a movable feature, and wherein the first feature is disposed on one of the plurality of walls of the infant care station, and wherein the second feature comprises a fixed feature.

8. The system of claim 1, wherein the third feature is selected from a group consisting of a movable feature, and a fixed feature.

9. The system of claim 1, wherein the state of the infant care station is determined using a state space model, and wherein the state space model uses a plurality of inputs indicating a plurality of positions of the first feature, the second feature, and the third feature.

10. The system of claim 1, wherein the instructions comprise an infant care station interface for obtaining the first spatial relationship and the second spatial relationship, and wherein determining the state of the infant care station comprises using a machine learning model that is trained to classify the state of the infant care station based on training data comprising a plurality of spatial relationships corresponding to the first spatial relationship and the second spatial relationship.

11. The system of claim 1, comprising a stereoscopic lens, wherein the image sensor is configured to capture a second image from a different angle than a capture angle of the first image, and wherein the first spatial relationship and the second spatial relationship are determined based on a point cloud representing the infant care station, and wherein the instructions are executable by the computer processor to generate the point cloud based on the first image and the second image.

12. The system of claim 1, wherein the instructions are executable by the computer processor to generate a segmented first image by segment the first image, and wherein the segmented first image comprises a plurality of representations corresponding to the first feature, the second feature, and the third feature, and wherein the first spatial relationship and the second spatial relationship are based on the segmented first image.

13. The system of claim 1, wherein the image sensor is disposed at a location selected from a group consisting of within the walls of the infant care station, at a head of the infant care station, outside the walls of the infant care station, and at a level that is even with a cover of the infant care station.

14. The system of claim 1, wherein the infant care station is selected from a group consisting of an infant incubator and an infant warmer.

FIG. 1

FIG. 2

FIG. 3

FIG. 4-1

400A-2

406

402

402

404

402

404

400B-2

406

402

402

404

402

404

400C-2

406

402

402

404

402

404

FIG. 4-2

FIG. 5-1

FIG. 5-2

600

Capture a First Image of a Portion of an Infant Care System — 602

Segment the First Image to Generate Representations of 1st, 2nd, and 3rd Features of the Infant Care System — 604

Determine a 1st Spatial Relationship Based on the 1st and 2nd Features — 606

Determine a 2nd Spatial Relationship Based on the 1st Feature and the 3rd Feature — 608

Determine a State of the Infant Care System Based on the 1st Spatial Relationship, the 2nd Spatial Relationship — 610

FIG. 6

FIG. 7

FIG. 8

Europäisches
Patentamt

European
Patent Office

Office européen
des brevets

# EUROPEAN SEARCH REPORT

**Application Number**

EP 25 19 8661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/108255 A1 (KHAIR MOHAMMAD [US] ET AL) 4 April 2024 (2024-04-04) * paragraphs [0061] - [0063], [0166] - [0171] * ----- | 1-14 | INV. A61G11/00 |
| A | GB 2 494 992 A (GEN ELECTRIC [US]) 27 March 2013 (2013-03-27) * page 6, line 17 - page 7, line 13 * ----- | 6 | |

**TECHNICAL FIELDS SEARCHED (IPC)**

A61G

The present search report has been drawn up for all claims

| Place of search | Date of completion of the search | Examiner |
|---|---|---|
| The Hague | 16 January 2026 | Kroeders, Marleen |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
..................................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 19 8661

This annex lists the patent family members relating to the patent documents cited in  the above-mentioned European search report.
The members are as contained in the European Patent Office EDP file on
The European Patent Office is in no way liable for these particulars which are merely given for the purpose of information.

16-01-2026

| Patent document cited in search report | Publication date | Patent family member(s) | Publication date |
|---|---|---|---|
| US 2024108255 A1 | 04-04-2024 | NONE | |
| GB 2494992 A | 27-03-2013 | DE 102012108795 A1 | 21-03-2013 |
| | | GB     2494992 A | 27-03-2013 |
| | | JP     6066637 B2 | 25-01-2017 |
| | | JP   2013066704 A | 18-04-2013 |

EPO FORM P0459

For more details about this annex : see Official Journal of the European Patent Office, No. 12/82